# EUROPEAN PATENT APPLICATION

(11) **EP 1 323 424 A1**
(43) Date of publication of application: **02.07.2003**
(21) Application number: 01130614.9
(22) Date of filing: 27.12.2001
(51) Int. Cl.: A61K 31/7032, A23L 1/09, A23C 21/04, C07H 15/10, A61P 29/00

(54) **Buffalo milk gangliosides**

(71) Applicant: SOCIETE DES PRODUITS NESTLE S.A., 1800 Vevey (CH)
(72) Inventor: Colarow, Ladislas, 1073 Savigny (CH); Turini, Marco, 1066 Epalinges (CH); Berger, Alvin, 1025 St. Sulpice (CH)
(74) Representative: Wavre, Claude-Alain

(57) **Abstract**

The present invention relates to gangliosides derived or isolated from buffalo milk, skimmed buffalo milk, buffalo milk serum or derivatives of either. Buffalo milk is reported to comprise gangliosides that are not contained in bovine milk, such as gangliosides that belong to the GM1-class. Furthermore, bufalo milk is found to comprise unknown gangliosides, denoted herein as ganglioside "F" and "L". Furthermore, the invention reports that gangliosides are surprisingly found in fractions of isolation procedures that were so far not considered to comprise gangliosides. Finally, milk or milk serum from buffalo, for example as derived from mozzarella cheese production, contains specific gangliosides in the same amounts as human breast milk, which makes it suitable for humanisation of infant and other formulas.

## Description

The present invention relates to gangliosides (GS) obtainable or derived from buffalo milk or derivatives of it. It relates further to a composition enriched with GS and the use of buffalo milk or a derivative thereof as a source of GS. Then the invention relates to the use buffalo milk or derivatives thereof to humanise nutritional formulas, or for isolation of GS that are functionally equivalent to GS occurring in humans. The invention also relates to GS that are found in the lower phase according to Folch.

### The Background Art

Gangliosides (GS) are different classes of sphingolipids that contain one to several sialic acid moieties. Each GS class includes species that mostly differ from each other by the nature of their ceramide moieties. The backbone in GS is a hydrophobic acyl sphingosine (that is, ceramide) moiety, to which are attached hydrophilic oligosaccharide units containing sialic acid. The amino group in sphingosine species is acylated with a fatty acid, in which the chain length and unsaturation degree are the main variables. In the case of a cerebroside, the primary hydroxy group is substituted by one sugar only (usually glucose or galactose). With the more complex GS, the terminal hydroxy group is substituted with an oligosaccharide. Typically, the oligo-saccharide chain of GS contains a sugar amino-acid known as sialic acid, which is an *N*-acetylated derivative of neuraminic acid. Hence sialic acid and *N*-acetyl-neuraminic acid (NANA) describe the same GS moiety. In a class of GS known as hematosides, their oligosaccharide chain is characterized by the absence of galactosamin.

An example for a known ganglioside with one sialic acid moiety is GM1. In this monosialo-ganglioside, the oligosaccharide chain consists of the following moieties: 1 glucose (Glc) unit, 2 galactose (Gal) units, an *N*-acetyl-galactosamin (GalNAc) unit and one *N*-acetyl-neuraminate (NAN). In the form: Ceramide-Glc-β1,4-Gal-β1,4-GalNAc-β1,3-Gal, wherein the first Gal is further connected α2,3 with NAN.

GS are abundant in the plasma membrane of nerve cells and other mammalian cells. Recently, the importance of these glycolipids has been more widely acknowledged. They interact with a large variety of biologically active factors, they have been postulated to play a role in signal transmission, cell-to-cell communication and to function as receptors.

For example, certain GS are reported to bind to cholera toxin as well as certain rotavirus particles. Thus, they may reduce the negative impact or symptoms of several enterotoxic bacteria and toxins,and reduce infections of humans. GS are reported to bind to various kinds of toxins, viruses and bacteria, including, but not limited to, *Helicobacter pylori, Escherichia coli* labile enterotoxin, shiga-like toxins, tetanus toxi, *Pseudomonas aeruginosa* lectin, and HIV-1 gp120 surface envelope glycoprotein.

It was also shown that feeding sphingolipids inhibits colon carcinogenesis, reduces serum LDL (Low Density Lipoprotein) Cholestrerol and elevates HDL (High Density Lipoprotein) Cholesterol.

In addition, there is evidence that the ganglioside GM1 might have neurotrophic-like activity and facilitate the recovery of brain neurochemical, morphological and behavioural function after an injury or during aging.

JP 05279379 (SNOW BRAND) GD3 (a disialic acid ganglioside) isolated from bovine milk is disclosed. However, bovine milk contains only trace amounts of GM1, a ganglioside present in human milk.

According to EP 463614 GM1 may be isolated from cerebral bovine tissue. Not only is this an utterly inconvenient way of acumulating GS, it must further be avoided due to a potential contamination with BSE (Bovine Spongiform Encephalopathy) and vCJD (variant Creutzfeldt-Jakob Disease) causing prions.

GS are also reported to be purified from pig brain. Such GS have been added to infant milk formulas (Rueda-R. et al., *Addition of GS to an adapted milk formula modifies levels of fecal Escherichia coli in preterm newborn infants*, J. Pediatr., 1998:90-94). Although these GS have the advantage that they are partly similar and functionally equivalent to certain human GS, their porcine origin is objectionable in food and drug products distributed worldwide.

Therefore, a need exists for a new source of GS. In particular, there is a need for a source of GS, which is naturally rich in GS. Moreover, the source should preferably provide GS that are similar or functionally equivalent to GS present in human milk.

There is a need for a GS source that is easily available, nutritionally safe, that has biological properties similar to those found in human breast milk and from which GS may easily be extracted.

There is further a need for new GS with beneficial effects so far not described.

The present invention addresses the problems set out above.

### Summary of the Invention

Surprisingly, it is found that buffalo milk and human milk have comparable GM1 and polysialo-ganglioside levels.

Remarkably, a side product of Italian cheese production (buffalo mozzarella and ricotta), that is decaseinated milk serum, contains surprisingly high levels of GS. This is especially so after a simple delactosing procedure.

Furthermore, Italian buffalo milk derived GM1 species bind cholera toxin. GM3 species from the same source bind rotavirus particles.

Moreover, lipophilic GS isolated from Italian buffalo milk mediate anti-inflammatory effects.

Consequently, in a first aspect the present invention provides GS, characterised in that they are obtainable, obtained or derived from buffalo milk, skimmed buffalo milk, buffalo milk serum, and/or derivatives of either.

In a second aspect the invention provides a composition enriched with GS, wherein the composition comprises fractions isolated from buffalo milk.

In a third aspect the invention provides the use of buffalo milk, skimmed buffalo milk, buffalo milk serum and/or derivatives of either as a source of GS.

In a forth aspect, the present invention provides the use of buffalo milk, skimmed buffalo milk, buffalo milk serum and/or a derivative of either to humanize infant or nutritional formulas or for isolation of GS that are functionally equivalent to GS occurring in humans.

In a fifth aspect, the present invention provides GS, characterized in that they are present in the lower organic phase (LP) according to Folch and in that they are not present in the upper aqueous phase (UP). In an embodiment, these GS are present in the UP, but not predominately. That is, they consititute less that 50% by weight of the sialic acid of the GS class.

An advantage of the present invention is that it provides an easily available source with unexpectedly high amounts of GS.

Another advantage of the present invention is that it provides sufficient amounts of GS that belong to GS classes also occurring in humans.

Yet another advantage of the present invention is that it provides GS that are functional equivalents of GS occurring in humans.

Still another advantage of the present invention is that it provides so far unknown GS.

It is in addition, a great advantage to provide useful GS derived from buffalo milk, because buffalo milk is rich in various valuable nutrients. Buffalo milk fractions may directly be used in milk-based formulas, such as infant formulas. In so doing, a double advantage is obtained: valuable proteins and lipids are added at the same time as the beneficial, often immunoactive GS present in high amounts in buffalo milk.

The abbreviations GM, GD and GT and their subscripts M, D, and T refer to mono-, di- and trisialo-GS, respectively. The terms therefore reflect the number of sialic acid moieties in an individual GS class as suggested by Svennerholm-L [(1963) *Chromatographic separation of human brain gangliosides*, J Neurochemistry 10:613-623].

In the drawings,

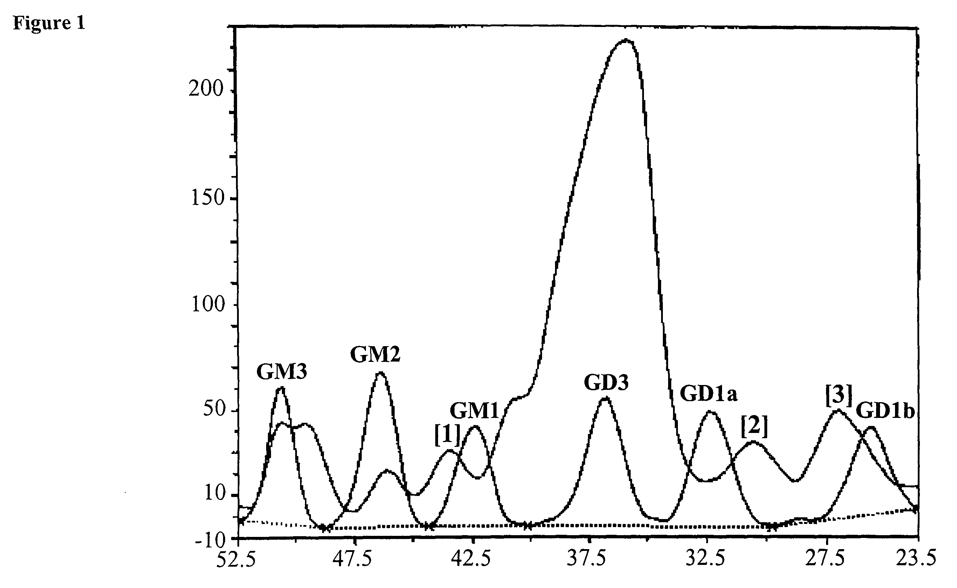

Figure 1 shows a mono-dimensional HPTLC densitogram showing equi-weight amounts (333 ng) of standard gangliosides (GM3, GM1, GD3, GD1a, and GD1b), so as GS isolated from Pakistan buffalo's mature milk. Peaks Nr. 1-3 from milk are thought to represent GM1 [1], *O*-acetyl-GD3 [2] and GT [3], respectively. The horizontal axis indicates elution distance in mm, starting from the right end of the axis. The vertical axis indicates the densitometric signal.

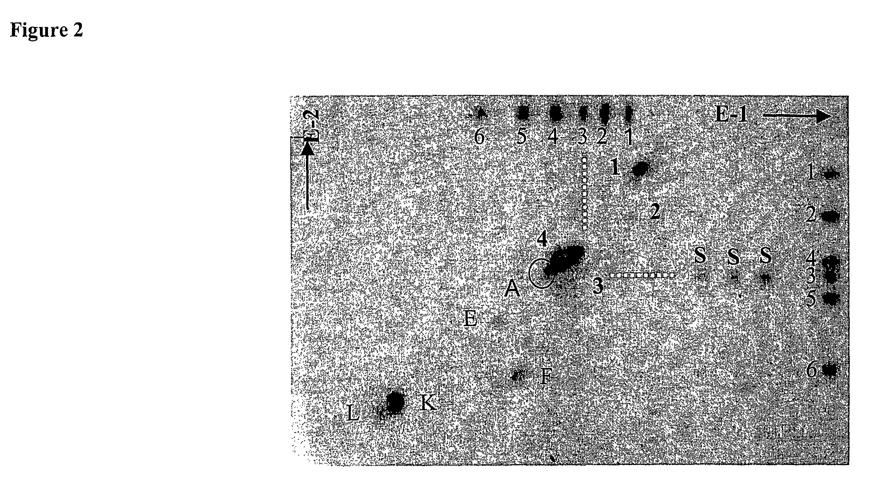

Figure 2 shows a bi-dimensional HPTLC of GS (4 nmol) isolated from Italian buffalo milk. The top and extreme right of the HPTLC plate show six standard GS classes. Abbreviations:
   1 = GM3,
   2 = GM2,
   3 = GM1,
   4 = GD3,
   5 = GD1a,
   6 = GD1b,
   A = unknown (see description),
   E = GT-1a,
   F = branched GM,
   K = branched GT,
   L = unknown GS.

S = bovine brain GM1 (64, 96 and 128pmol) deposited on the plate as an external standard before elution in the 2^{nd} direction .

Elution causes lipophilic GS to move to the right and the top of the plate, whereas hydrophilic GS will remain in the bottom left comer. The most hydrophilic GS is marked as "L" in Figure 2.

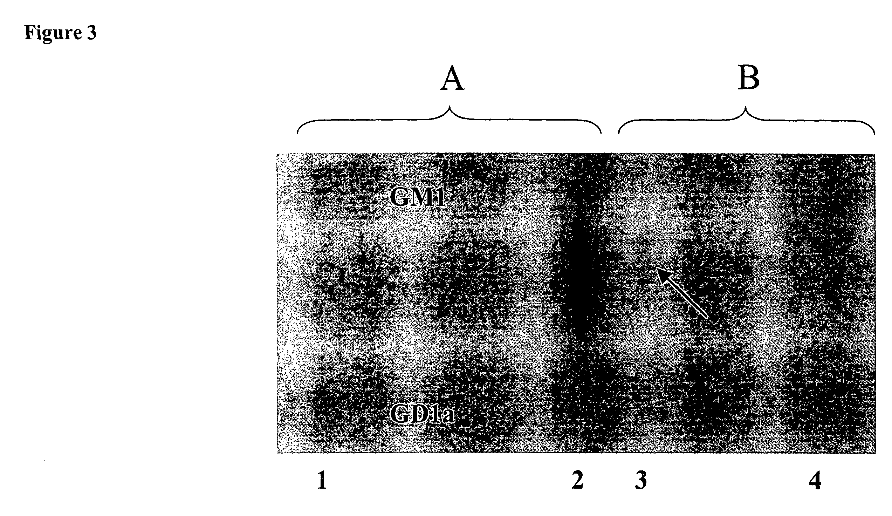

Figure 3 shows a TLC-overlay assay using cholera toxin subunit B (CTB). Two series of the same GS samples were separately deposited and chromatographed on the same TLC plate. The plate was cut to two halves "A" and "B" to separately visualize each of the two sample series. "A" was treated with an anisaldehyde reagent to detect all GS classes, as shown in a magnified section of the chromatogram "A". Lane 1 shows the detected spots of standard GM1, GD1a from bovine brain. Lane 2 on the same section shows GS isolated from buffalo milk serum that includes GM1. The presence of GM1 was established in the second sample series in "B" as follows.
GS in "B" were immobilized and exposed to CTB. Unbound CTB in "B" was washed off. GS-bound CTB was detected in "B" using a chlorophenol reagent. Lanes 3 and 4 show the chromogenic response of GM1 from buffalo milk and bovine brain, respectively, after the CTB- and chlorophenol-based detection procedures

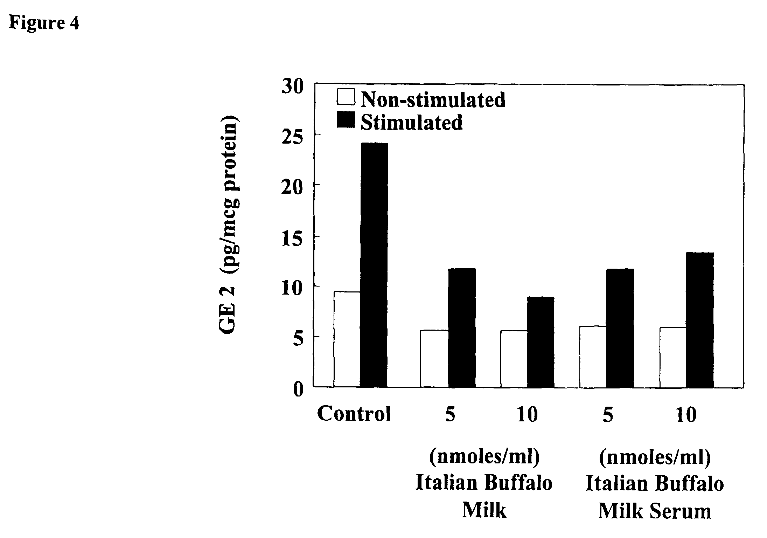

Figure 4 shows the PGE-2 secretion by an intestinal cell line challenged with TNFα. Filled boxes indicate the PGE-2 secretion by cells that were exposed, 30h prior to treatment with TNFα, to different concentrations of GS (5 and 10nmol/mL) isolated from buffalo milk or buffalo milk serum. Clear boxes were not challenged with TNFα. The addition of GS decreased significantly the PGE-2 response of the intestinal cells *in vitro.* The boxes of the control demonstrate the intensity of the PGE-2 response to TNFα treatment, without application of GS.

### Detailed Description of the Invention

Within the context of this specification the word "comprises" is taken to mean "includes, among other things". It is not intended to be construed as "consists of only".

In the context of the present invention, the term composition is intended to encompass any nutritionally complete or supplementary consumable product. Hence, the composition may be consumed by humans, pets, such as cats and dogs, for example, and/or other animals. It may, be a bar, a snack, a nutritional formula, an infant or baby formula, an ice cream, a dairy product, a confectionery product, or it may be a supplement or a medicament, which may, optionally, be added to another food product, such as the ones given above. It may also be a liquid product.

Further, in the context of the present invention, the term GM1 refers to a class of monosialo-GS species, which, if separated on a bi-dimensional HPTLC using acidic and alkaline mobile phases, migrate most closely to the GM1 species isolated from bovine brain. Hence, the class of GM1 reported herein, and the class of GM1 from bovine brain have comparable chromatographic behavior, although their individual molecular species may carry different ceramide moieties.

All terms comprising the name of "Folch", such as "Folch's upper phase" and Folch's lower phase" refer to a defined method for the isolation of GS as described in Folch-J, Less-M and Sloane-Stanley-GH [(1957) *A simple method for the isolation and purification of total lipids from animal tissues,* J Biol Chem 226: 497-509]. It will be clear to the skilled person, that the organic solutions described therein to extract and purify specific lipids and perform a separation are not the only ones possible to isolate GS. Similarly, the specific ratio of solvents as chloroform-methanol or chloroform-methanol-water as mentioned in this reference may be substituted or modified and still be suitable to resolve GS. Hence, the term "Folch's solution" and the like are also considered to cover functional equivalents of the solvents and other ways of isolation of GS.

The term "Folch's upper phase" or "upper phase according to Folch", also abbreviated as UP, and the like do more specifically refer to the aqueous or hydrophilic phase, which is described in the Folch reference. Generally, in the upper phase the approximate proportions of chloroform, methanol and water are 3:48:47 by volume (v:v).

The term "Folch's lower phase" (LP), accordingly, does refer to the organic or lipophilic phase described in this reference. Generally, the approximate proportions of chloroform, methanol and water in the lower phase are 86:14:1 (v/v).

The term "derivative" of buffalo milk, skimmed buffalo milk and/or buffalo milk serum refers to any product or isolate obtainable on the base of these sources. For example, derivatives would include hydrolysed and/or fermented products. Another example may be vegetable oil-filled milk, that is milk in which all or part of the lipids are replaced by vegetable or other oils. Nutritional and/or infant formulas comprising the above mentioned sources provide still another example of the term "derivative". Likewise, dried and/or reconstituted buffalo milk serum, and milk fractions (see below) are considered as derivatives.

The term "milk fraction" is to be construed as to comprise, amongst others, milk fractions known from the dairy industry and fractions appearing in cited literature or the procedures set forth herein. Skimmed milk, delactosed milk, milk serum, acidic and acid whey, acidic lipid fractions, Folch's upper and lower fractions serve as few examples for milk fractions.

In an embodiment, the GS according to the present invention are isolated GS.

In a preferred embodiment of the present invention, the buffalo milk serum or the derivative of it is buffalo milk serum obtained from mozzarella or ricotta cheese production. Preferably, it is delactosed milk serum.

In a preferred embodiment, the GS according to the present invention are functional equivalents from GS present in human tissues and/or secreted fluids including milk.

Preferably, the GS according to the invention belong to GS classes present in humans. For example, they may be present in human milk or human nervous tissues such as human brain.

Preferably, the GS according to the invention are GM, GD and/or GT.

In a preferred embodiment, the GS according to the present invention are present in or isolated from the upper aqueous and/or the lower organic phase according to Folch.

In contrast to previous reports, the lower organic phase (LP) of the Folch purification method described below also contains substantial amounts of specific GS.

Folch et al (1957) showed that pure lipids partitioned into the organic LP while non-lipids and other contaminants remain in the aqueous UP. The Folch's method was reviewed by Hakomori-S [(1983) Chemistry of glycosphingolipids, Chapter 1, in Sphingolipid Biochemistry (Hanahan-DJ; Editor) Plenum Press, New York, pages 1-165]. Based on published data, the author states that the partition pattern of glycosphingolipids in the UP and LP varies greatly depending on the coexisting lipids, and the concentration of ions. His scheme suggests that sialosylgalactosylceramide (a minor GS class GM4, which belongs to hematosides due to the absence of hexosamin) and 10 to 20 % of GM3 collect in the LP.

This shows clearly that up to date, it was thought that GS extracted from biological tissues or milk would collect predominantly in the aqueous UP according to the Folch's partition. It was, however, unexpectedly found that the lower, lipophilic phase, may comprise as much as 40% of all GS present in buffalo milk. This is surprising, because GS form micelles that are in general pseudo-soluble in water and in the aqueous UP.

In a further embodiment, the GS according to the present invention belong to the classes of GM1, GM2, GM3, GD3, GT1a, GT1b, branched GT-K and/or unknown GS denoted as "GS-L" or branched "GM-F".

The GS of these classes may include several different GS species. For example, the GS class of GD3 was shown to comprise different species (individual molecules). It was shown, in the context of the present invention, that GD3 species isolated from the organic LP according to Folch bound to nar-3, a rotavirus toxin. GD3 isolated from the aqueous UP did not bind to this toxin.

The GS-classes of GS present in buffalo milk are assigned according to their migration patterns in mono- or bi-dimensional HPTLC. Figure 1 and 2 show these patterns that, in certain cases coincide with those of available GS standards. Some of the GS are attributed to GS that were described by other authors already, however not from buffalo milk.

Hence, the GS assigned "K" in Figure 2 may be the branched GT described in a paper of Rueda-R, Maldonado-J and Gil-A [(1996) *Comparison of content and distribution of human milk gangliosides from Spanish and Panamanian mothers,* Ann Nutr Metab 40: 194-201].

The GS denoted "F" in Figure 2 corresponds most likely to a branched GT described in Takamizawa-K, Iwamori-M, Mutai-M and Nagai-Y [(1986) *Gangliosides of bovine milk,* J Biol Chem 261: 5625-5630].

The GS denoted "L" in Figure 2 was not attributed to any known GS class, hence it is a novel GS. Its migration pattern similar to that of GT-K suggests that an increased presence of hydrophilic moieties lowers the migration velocity of GS "L" compared to GT-K.

Other GS present in buffalo milk were identified more clearly as is given in the description of the figures.

In a still further embodiment, the GS according to the present invention exhibit a mono-, or bi-dimensional HPTLC migration pattern that allows or suggests allocation of the GS to at least one GM, GD or GT occurring in human milk.

Preferably, the GS according to the present invention are used as a medicament.

In still another embodiment, the GS according to the present invention are used as a composition and/or supplement in food products, instant milk, infant and/or other nutritional formulas, and/or pet food. The GS may used in foods for agricultural species, too.

In a preferred embodiment, the GS according to the present invention are used as an anti-inflammatory agent.

In another preferred embodiment, they are used as a binding and/or neutralizing agent of infective and/or toxic agents. For example, the GS according to the present invention are used to bind and/or neutralize tetanus toxin, rotavirus toxins nar-3 and/or RRV, Cholera toxin subunit B, HIV-1 gp120 surface envelope glycoprotein, Shiga-like toxins, for example.

Preferably, the GS according to the invention are used as an agent to prevent and/or treat symptoms and/or infections originating from pathogenic organisms.

Examples for pathogenic organisms include, rotaviruses, HIV-1, HIV-2, *Vibrio cholerae, Helicobacter pylori*, uropathogenic *Escherichia coli,* tetanus, *Pseudomonas aeruginosa,* and/or *Chlostridium botulinum.*

In particular, in and embodiment, the GS of the present invention are used as an agent to reduce or treat symptoms of cholera and/or rotavirus infections.

Preferably, the GS are used as a cholera toxin binding agent or as an agent binding to rotavirus particles.

Preferably, the GS according to the invention are used to treat or prevent reduced function of neuronal tissue or cells due to aging, age related diseases or injury.

Age related disease include, for example, Parkinson's and/or Alzheimer's disease.

For example, the GS according to the present invention may be used as an agent to regulate neurotransmitter function.

Preferably, the GS are used as an agent to enhance the choline acetyltransferase and/or tyrosine hydroxylase activity in mammals.

For example, the present invention provides the use of buffalo milk, skimmed bufalo milk, buffalo milk serum and/or a derivative of either in the preparation of a medicament or a nutritional composition to
- reduce symptoms related to cholera and/or rotavirus infections
- treat or prevent Alzheimer's and/or Parkinson's disease
- enhance the choline acetyltransferase and/or tyrosine hydroxylase activity
- enhance the synthesis of acetylcholine and/or dopamine, or,
- treat or protect against retinal damage after injury or due to aging.

More preferably, the GS are used as an agent to enhance the synthesis of acetylcholine and/ or dopamine in mammals.

The GS according to the invention, may further be used to prevent, treat or protect against retinal damage after injury or due to aging.

The variety of different GS present in buffalo milk may be isolated, purified or enriched in any adequate way. The literature presents a multitude of methods concerned with extraction of GS from different sources. The extraction method described below is understood to be just one possible way of extracting GS. It is exemplary because literature concerning specific steps are described and the necessary equipment is commercially available. Furthermore, the method below allows quantitative analysis of different components of buffalo milk.

In contrast to this, it is mentioned that most applications of GS derived from buffalo milk or derivatives thereof will not require a detailed isolation process. For many purposes, it is sufficient to use buffalo milk directly or a derivative thereof, for example, an acidic lipid fraction of buffalo milk, in order to achieve a GS-mediated effect. The amount of GS contained buffalo serum or a derivative thereof may already be sufficiently high. However, for the purpose of completeness, a complete process of isolation of specific GS classes is given below in an exemplary way.

Accordingly, buffalo milk, preferably from Italian or Pakistan buffalo, skimmed buffalo milk, buffalo milk serum, or derivatives of either may be directly used to administrate GS to an animal species, for example, a mammal. Examples of derivatives are dried and/or reconstituted buffalo milk serum, lipid fractions, such as Folch's UP and LP and their fractions, acidic lipid fractions and the like.

Generally, recoveries, in particular quantitative ones, of GS from milk, require laborious extraction and purification procedures for several reasons. First, high lactose levels compromise most analytical and isolation procedures. Second, the small amount of GS in milk requires large sample volumes, and further, GS are to be isolated from a complex lipid matrix.

Current methods are based on partitioning lipid extracts into Folch's UP aqueous phases (UP) and lower organic phases (LP) (see reference cited above). Most GS are thought to accumulate in the UP along with penta- and polyhexosylceramides. It is supposed, without desiring to be bound by theory, that the partition of GS may depend on weight ratios of their lipophilic to hydrophilic moieties, so as on specific presence of other complex lipids and ions. For instance, about 70% hematosides (*N*-acetyl-galactosamine-free GS) with highly lipophilic and elongated C20:0 to C24:0 fatty-acid moieties may collect in the organic LP. On the other hand, up to 80% hematosides with less elongated and lipophilic C16:0 to C18:0 fatty-acid moieties may collect in the UP.

Some authors used only the aqueous, GS-extraction phases for the isolation and analysis of GS in milk. Only minor amounts of GS (6-7% total sialic acid) were detected in the organic LP obtained from first-day bovine colostrum. The complete absence of measurable quantities of GS in the organic LP was even confirmed by some authors [Puente-R, Garcia-Pardo-LA, Rueda-R, Gil-A and Hueso-P (1996) *Seasonal variations in the concentration of gangliosides and sialic acids in milk from different mammalian species,* Int Dairy J 6: 315-322; (1992) *Changes in ganglioside and sialic acid contents of goat milk during lactation*, J Dairy Sci 77: 39-44].

In contrast to this and other references, it is one of the surprising findings of the present invention that certain GS are found in high levels in the lower organic and lipophilic phase (LP). Therefore, data from authors who used a different procedure for the isolation of GS from the LP than detailed later must be considered with caution.

For example, the staring point of purification may be buffalo milk or buffalo milk serum. In case of buffalo milk, this can be purchased readily where buffaloes are raised and buffalo milk is produced. For example, the main buffalo region in Italy is Campania between Naples and Foggia. Italian buffalo milk may be obtained from cattle breeders in this region. In 1999 Italy produced over 120'000 t buffalo milk per year. Buffalo milk production showed the highest 5-year average growth of more than 4% compared to overall milk production in the world (1 to 1.8 % growth).

In general, any buffalo milk may be used to isolate GS. For example, Pakistan or Italian buffalo milk or derivatives may be used. However, any buffalo species or breed may be suitable to isolate the GS according to the invention. In general, water buffalo or African buffalo milk may be used. Accordingly, milk from species of the genera Bubalina (*Bubalus* and *Anoa*) and Syncerina (*Syncerus*) is suitable. Preferably, milk from water buffalo (*Bubalus bubalis*), for example, Italian, Pakistan or Indian buffalo may be used. Very often, commercially available milk of buffalo, for example, from Pakistan, is "ccontaminated" with bovine milk, because buffaloes and bovine cattle are often kept together. However, also the buffalo milk comprising bovine milk is suitable in the context of the present invention, because it still comprises the biological properties described herein.

If Pakistan or Indian buffalo milk is used as source, such milk may be easily obtained from local breeders. Buffalo breeding is widespread in India, Pakistan and other countries.

Whatever buffalo milk source may be used, be it pure buffalo milk, buffalo milk serum or another derivative of buffalo milk, it may be advantageous to remove lactose from the liquid. For example, to obtain a concentrated source of GS. Delactosation of milk may be performed according to any suitable method. Generally, this may be done by laboratory-scale dialysis or, industrial ultrafiltration. For example, if milk is directly used, it may first be homogenized at 300 bar and 20°C. The homogenate may be lyophilized to determine total milk solids, and reconstituted with an appropriate volume of water and dialysed. At laboratory scale, for example, dialysis may be carried out in 3500-Dalton and 34-mm o.d. tubes (Spectra/Por®, Spectrum Medical Industries Inc, Laguna Hills, California) for 72 hours against running de-ionized and preferably sterilized water cooled to 4°C. The delactosed retentate may be lyophilized and weighted to determine non-lactose milk solids. In case of Italian buffalo, the non-lactose milk solids may account for about 70% of the total milk solids.

As a next step, the delactosed milk solids may be separated into their lipid and protein fractions. This may be done according to defined and appropriate methods. For example, the above-mentioned reference [Folch et al (1957)] is suitable to separate crude lipid extracts into their hydrophilic UP and lipophilic LP fractions, the both of which may contain GS. A protocol in the sense of Folch et al may be as follows.

Accordingly, one part of the delactosed and lyophilized retentate (25g, for example) may be suspended in two parts of water at 50°C and homogenized for one minute with 19 volumes chloroform-methanol 2:1 (volume:volume = v:v). The homogenate may be passed through a Büchner type filter fitted with a standardized paper membrane No. 602 (e.g. Schleicher & Schuell GmbH, Dassel, Germany). The resulting filtrate may be partitioned overnight against 0.2 volumes 0.88% (by weight) KCl at 20°C. Thereby, the upper aqueous (UP) and lower organic (LP) phases are usually formed, preferably in a separatory funnel.

The LP may be drawn off, vacuum-concentrated to dryness using a regular rotary evaporator at 50 to 65°C, and the resulting residue separated by ion-exchange chromatography to retain lipophilic GS in the acidic fraction. The UP containing hydrophilic GS may be concentrated to dryness as above, dialyzed to remove non-lipid contaminants, and further separated by ion-exchange chromatography as above.

The separation of non-acidic and acidic fraction from the UP and LP lipids may also be derived from the literature [Ledeen-RW, Yu-RK and Eng-LF (1973) *Gangliosides of human myelin: sialosylgalactosylceramide (G*_{*7*}*) as a major component*, J Neurochem 21: 829-839; Colarow-L and Traitler-H (1985) *The application of gradient saturation in unidimensional planar chromatography of polar lipids*, J High Resolution Chromatogr & Chromatogr Communications 8: 341-346)].

In this sense, the LP, after concentration and drying, may be re-dissolved in an increased volume (e.g. 500mL) of chloroform-methanol-water solution 30:60:9 (v:v) and passed through a glass column containing DEAE-Sephadex A-25™ (acetate form). The latter is supplied by pharmacia Fine Chemicals (Uppsala, Sweden). Thus, non-acidic and acidic lipid fractions may be separated. Non-acidic LP-lipids comprise usually acylglycerols, neutral phospholipids, glycolipids and unsaponifiables such as sterols and hydrocarbons. GS, sulfatides, free fatty acids and acidic phospholipids may generally be collected in the acidic-lipid fraction.

The acidic lipid fraction may be concentrated to dryness and re-dissolved in 5mL 0.6N sodium hydroxide in methanol, followed by 10mL chloroform. Glycerophospholipids are deacylated by gentle agitation of the solution for 60min at 37°C to yield free fatty acids and non-lipid moieties. This alkaline solution may be neutralized with 0.75mL 4N acetic acid, diluted with 10mL isopropanol that mediates an azeotropic removal of water, and vacuum-concentrated to dryness as above. The residue may be re-dissolved in 1mL water, pipetted into an appropriate dialysis tube (e.g. a 3500-Dalton 1-cm o.d. Spectra/Por tube from Spectra/Por®, Spectrum Medical Industries Inc, Laguna Hills, California) and the whole precedure may be repeated using 2 x 1mL water to avoid losses. The subsequent dialysis proceeds as above to remove salts and non-lipid contaminants in about 24h. The resulting dialysate may be lyophilized using a conical glass flask. The GS containing lyophilisate may be further purified by Unisil® chromatography (see below).

Similarly, the GS-containing acidic fraction of the UP obtained according to the Folch method (see above) may be separated. Hence, the UP may be vacuum-concentrated to 5-10mL and taken in 4 x mL water for a 48h dialysis as above. This dialysis generally removes non-lipid contaminants such as residual lactose, polypeptides and salts. The resulting retentate containing crude GS along with contaminant sulfatides, trace phospholipids and other materials, may separately and successively redissolved in 1 x 4 and 2 x 2mL chloroform-methanol-water 30:60:8 (v:v) and loaded onto a 15 x 1.5cm o.d. glass column containing DEAE-Sephadex A-25® in acetate form (5-mL bed volume) as above. In the case of the UP, quartz sand (Cristobalit®, Siegfrid Nr.20953802) may be added on the top of the chromatographic sorbent. The sample may be allowed to pass through the column and neutral lipids may be eluted from the column with 3 volumes chloroform-methanol-water 30:60:8 (v:v). The eluate may be vacuum-concentrated to dryness as above. Acidic lipids that include GS may be eluted from the same column using 5 volumes chloroform-methanol-0.8 N sodium acetate (30:60:8, v:v). The resulting eluate may be vacuum-concentrated to dryness and re-dissolved in water and dialyzed for 24 hours in a Spectra/Por® tube against running deionized water at 4°C as above. The dialysate may be lyophilized to yield a GS-containing acidic upper phase fraction that may be further purified.

The lyophilized acidic lipid fractions isolated from the aqueous UP and organic LP (see above) may then be further purified to remove sulfatides and non-lipid contaminants. Such process was described before by Yu-RK and Ledeen-RW [(1972) *Gangliosides of human, bovine and rabbit plasma,* J Lipid Res 13: 680-686].

The acidic fraction may be further purified using Unisil™ columns, for example. In particular, About 350mg Unisil® 200-325 mesh (Clarkson Chem. Co. Inc., Williamsport, Pennsylvania) sorbent may be used to prepare a suitable column. A Celite® filter aid (Celite® Nr. 545) may be added to the column to form a thin layer on the top of the sorbent. This last step may not be necessary for purification of the LP acidic lipids. The desalted, acidic UP lipids may be dissolved in 3 x 1mL chloroform-methanol 8:2 (v:v) and completed with about 20mg Celite ® Nr. 545. The resulting slurry (i.e. each time 1mL) may be successively loaded onto the Unisil® column. Neutral lipids and sulfatides may be eluted with 10mL chloroform-methanol 8:2 (v:v). GS may be eluted using chloroform-methanol 2:3 (v:v). The GS eluate may be concentrated to dryness, weighted, and re-dissolved in 120:60:9 chloroform-methanol-water solution. The solution containing pure GS may then be stored at -20°C, for example.

From 2 kg Italian buffalo milk, finally 1 to 2mg, preferably 1.2 to 1.5mg pure lipophilic GS and 3 to 5mg, preferably 4.0 to 4.5mg hydrophilic GS may be obtained.

In further steps, the isolated GS mixture obtained from buffalo milk, buffalo milk serum or another derivative of buffalo milk, may be separated into their different GS classes. For example, this may be done by high-performance thin-layer chromatography (HPTLC) of GS. For specific GS classes, a planar HPTLC may not be sufficient and a bi-dimensional HPTLC is preferably performed. The latter method may also be adequate to identify unknown GS classes, because elution of samples may take place in two (i.e. X- and Y-axis) directions sequentially. The X-axis elution may be performed by using an acidic-like mobile phase, whereas an alkaline mobile phase may be used for the Y-axis elution. Since GS are acidic lipids, they migrate differently in acidic and alkaline mobile phases. For instance, this may be derived from in Figure 2 showing a reversion in the elution order of GM1 and GD3 in the X-axis elution, to GD3 and GM1 in the Y-axis elution. This bi-dimensional migration pattern may be particularly useful as one of the fastest methods to tentatively or preliminary identify GM1.

Tentative or preliminary identification of most GS may advantageously occur as above and in combination with the use of referencial GS standards. Mono, di- and certain polysialo-GS standards are commercially available. For example, such standards from bovine brain supplied by Lucerna Chem AG (Lucerne, Switzerland) may be used.

In the case of bi-dimensional HPTLC, GS samples may be separated as follows. A HPTLC glass chamber (for example, from Camag AG, Muttenz, Switzerland) fitted with a saturation paper that opposes the elution may be allowed to equilibrate with a suitable mobile phase. Generally, a silica gel plate is cleaned in both the X-axis and Y-axis directions using successively 1:1 (v:v) chloroform-MeOH as the mobile phase. A sample (for example, comprising UP and LP GS as isolated according to the above procedure) may be loaded together with a standard 1, such as shown in Figure 2. Elution in a first direction (X-axis) may take place with a solvent 1. After elution, the plate may be dried for 2 hours under N₂ at 20°C. Thereafter, a second standard 2 may be loaded and a second elution with solvent 2 in the other direction seen as Y-axis in Figure 2 (in a right angle to direction A) may be conducted. Suitable solvents for the X- and Y-axis directions and other equipment may be derived from the literature [for example, Ledeen-RW and Yu-RK (1982) *Gangliosides: structure, isolation, and analysis,* Meth. Enzymol. 83: 139-191; Zanetta-JP, Vitiello-F and Vincendon-G (1980) *Gangliosides from rat cerebellum: demonstration of considerable heterogeneity using a new solvent for thin layer chromatography*, Lipids 15: 1055-1061].

After elution, the chromatogram may be flushed with a stream of N₂ to remove solvents.

Visualization of GS on the silica plates may be achieved with any sialic-acid specific detection reagent.

For example, a spray reagent may be prepared comprising resorcinol (recrystallized from benzene). The spray reagent may be prepared by dissolving 2g resorcinol in 100mL water to form a stock solution. Ten mL of this stock solution may be added to 80mL concentrated HCl containing 0.25mL copper sulfate solution (0.1M), and the total volume brought to 100mL with water. This resorcinol reagent may be allowed to stand for 4h at room temperature and then stored at 4°C.

The spray reagent may then be sprayed onto the HPTLC plate as a fine mist to avoid an excessive saturation of the sorbent. A suitable spraying device designed for HPTLC may be selected to this end. The freshly sprayed chromatogram may be covered with a clean glass-plate pre-heated to 100-120°C and exposed to elevated temperatures (120°C) to obtain a GS-specific chromogenic response.

Resorcinol-detected GS usually appear as polymorphic blue-tinted spots against a white background. Alternatively, an anisaldehyde spray-reagent may be used to detect GS.

If Italian buffalo milk is used to isolate specific GS, the bi-dimensional HPTLC will show a ganglioside that has a similar migration pattern as GM1 from bovine brain. Hence, one or several species of the GS class GM1 are present in buffalo milk.

In addition, GT K, a GS known from human milk is detected in Italian buffalo milk. GT K was reported in human milk by Rueda-R, Maldonado-J and Gil-A [(1996) *Comparison of content and distribution of human milk gangliosides from Spanish and Panamanian mothers,* Ann Nutr Metab 40: 194-201)].

Furthermore, an unknown GS with a similar migration pattern as GT K described above is present in Italian buffalo milk.

The fractions isolated by HPTLC may be further quantitatively analyzed. The skilled person is aware of such procedures, which may consist of determination of lipid-bound sialic acid, for example that present in individual GS classes as separated by HPTLC. Concentration of sialic acid may be determined by colorimetry using a pure sialic-acid solution of known concentration as a calibration standard.

Isolated GS may directly be applied in consumable products, food products, beverages, nutritional formulas, infant formulas and the like.

Alternatively, specific buffalo milk fractions may be used to furnish GS. For example, delactosed and dried buffalo milk serum may be added to formulas directly.

Buffalo milk serum, for example, is a particularly suitable source of GS. One advantage with the use of Italian buffalo milk serum to obtain GS is that the serum appears in high amounts as an inexpensive waste product of buffalo mozzarella cheese production. GS found in buffalo milk are at least partially also found in the serum, for example, GM1. By one single operation, that is, delactosing the serum, remarkably, a source highly enriched in GS is obtained, which may be directly applied to other products.

Very useful, for example, is the serum that arises as a waste product in the mozzarella or ricotta cheese production. This serum is commercially available for a reasonable price and may already be deprived of substantial milk proteins or fats, and hence, has a much higher ratio of GS with respect to total dry matter.

For example, 15 to 20 g delactosed and powdered Italian buffalo milk serum equates in lipid-bound sialic acid to 1L in human milk. Hence, the delactosed powder may be directly used in adequate amounts in instant, nutritional and/or infant formulas, for example. The delactosed and powdered serum from buffalo milk mozzarella or ricotta production is an industrially very advantageous source or delivery system of GS, moreover of GS classes that are also found in human milk.

Therefore, powder from delactosed Italian milk serum may be directly used as a medicament in various applications or added to food products. Infant formulas provided with such serum may directly humanized thereby, since the GS profile of the formula will correspond more closely to the one of human milk.

Apparently, also other fractions from buffalo milk may be used for the same end. For example, upper or lower Folch fractions may be used as medicaments or supplements of food products and formulas.

The following examples are given by way of illustration only and in no way should be construed as limiting the subject matter of the present application. Percentages and parts are by weight unless otherwise indicated. All percentages are by weight, unless otherwise indicated.

Ratios of different liquids present in a mixture of solvents and/or water, as chloroform, methanol and water, or, chloroform and methanol, for example, are given as volume:volume (v:v), unless otherwise indicated.

### Example 1: Isolation and Identification of GS from Pakistan Buffalo Milk.

The purpose of this experiment is to isolate, identify and quantify GS from Pakistan buffalo milk.

### MATERIAL AND METHODS

Briefly, the isolation procedure comprises delactosing milk, extracting lipids by the Folch method, dialysis of the upper and the lower Folch phases, which contain hydrophilic and lipophilic GS, respectively. This procedure is completed by deacylation of glycero-phospholipids present in the lipophilic lower phase, ion-exchange chromatography of acidic lipid fractions and, Unisil ® purification of GS. The detection and quantification of individual GS classes is performed by planar chromatography and scanning densitometry.

Milk samples were collected and lyophilised from Pakistan buffalo. Samples included milk from healthy, normal animals (MB, milk liquid: 3025 g, solids: 427 g) and an animal kept on a rice growing area (RB, milk liquid: 3305 g, solids: 470 g).

### Dialysis

Lyophilised milk samples were reconstituted at 65°C with deionised water to 50% total solids (TS) and homogenised at 50°C and 180 bars (Büchi Homogeniser Nr. 196). The homogenate was quantitatively piped into a 50 x 3.4cm i.d. Spectra-Por® Nr. 132 725 tube (MW cut-off: 3500 Dalton). Residual milk was washed using about 2 x 10mL deionized water and passed through the homogenizer into the same tube. The homogenized milk was dialyzed for 65h at 4°C against deionized water. The resulting dialysates (delactosed milk) were lyophilized and stored frozen until analyzed.

### Folch's Partition of GS

The lyophilized-delactosed milk powder was briefly sonicated (47°C) with 50 ml water to obtain a homogenous suspension. The suspension was mixed (Polytron™) for 2min with 19 volumes (1425 ml) of chloroform-methanol 2:1. The homogenate was mixed with 2g Celite Nr. 545 (Merck No. 102693) and vacuum-filtered using a Büchner Nr. 602 filter also containing Celite (2g). The filtrate was poured into 2-L separatory funnel, diluted with 0.2 volumes 0.88-% KCl, vigorously agitated for 1min and then allowed to separate into its upper (UP) and lower (LP) phases overnight at 20°C. The UP was concentrated to 0.1 volumes and dialyzed at 4°C as above.

### Ion-Exchange Chromatography

Both the LP and UP were separated by ion-exchange chromatography into their non-acidic and acidic fractions. Briefly, the LP was concentrated to dryness, re-dissolved in choroform-methanol-water (C-M-W) 30:60:8 and passed through DEAE-Sephadex A-25® (acetate form) (Pharmacia Fine Chemicals) to obtain both non-acidic and acidic lipid fractions (the first contained acylglycerols, neutral phospholipids plus glycolipids and unsaponifiables. GS, sulfatides, free fatty acids and acidic phospholipids were collected in the acidic lipid fraction fraction). The acidic lipid fraction was concentrated to dryness, re-dissolved in choloroform-methanol (C-M) 2:1, its glycero-phospholipids deacylated using 0.6N NaOH for 1h at 37°C and neutralized (4N acetic acid). The neutralized solution was desalted on a Sephadex G-25 Superfine® (Pharmacia Fine Chemicals) column. The final desalted product was solubilized in C-M 8:2.

The UP dialysate was lyophilized, re-dissolved in chloroform-methanol-water 30:60:8 and separated into its non-acidic and acidic fractions as above (neutral glycolipids with more than 3 carbohydrate moieties collect in the non-acidic UP. GS, sulfatides and probably, minor amounts of acidic phospholipids collect in the acidic upper phase). The acidic upper phase was concentrated to dryness, deacylated and desalted as above. The final desalted product was solubilized in C-M 8:2.

### Colorimetry of Lipid-Bound Sialic Acid

GS isolated from the Folch UP and LP were separately dissolved in 5.0mL chloroform-methanol-water 120:60:9. From 200 to 500µL aliquots of these GS stock solutions were analyzed in glass test-tubes for their total lipid-bound sialic acid by colorimetry at a defined wavelength (λ=580nm). Pure sialic acid (Sigma No. A 9646) was used to calibrate the method for a 1- to 200-nmol range (i.e. 0.3093 to 61.86µg). Briefly, quantitative aliquots of pure sialic acid (1 to 200nmol) dissolved in water were made up to 2.0mL with water (alternatively, aliquots of the above GS stock solutions were desolvantized and dissolved in 2.0mL water). Each of the above samples and a blank (2.0mL water) were completed with a resorcinol reagent (2mL), tightly capped, and reacted for 15min at 100°C. The reacted solutions were cooled to 20°C, completed with 4mL n-butyl acetate plus 1-butanol (85+15; v+v), kept in ice-bath for 15min, and centrifuged for 1min at 1000g to separate each sample into its upper chromogenic and lower (discarded) phases. The chromogenic response of sialic acid was measured in the upper phase by colorimetry (λ=580nm) against blank. Accurate concentrations of sialic acid were calculated using the following calibration equation: *Sialic acid (in nmol) = 425.9*Optical Density - 0.0539.*

In addition, the same colorimetric method was used to analyze sialic acid in GS classes after their separation by unidimensional HPTLC and a non-invasive detection. In each case, HPTLC sorbent containing an individual GS class was scrapped off, brought to 2.0mL and analyzed by colorimetry as described above. Details on the unidimensional HPTLC are described below.

### Unidimensional HPTLC and Scanning Densitometry

A baseline separation of six commercially purchased bovine brain GS standards was obtained (Accurate Chem. and Sci. Corp, Westbury, New York). GS fractions isolated above were quantitatively separated on 20 x 10 cm Whatman HP-K plates (Cat. Nr. 4807-700, Madstone, UK) as 7-mm bands along with GS standards. Baseline resolution of six GS classes was obtained using methyl acetate-n-propanol-chloroform-methanol-0.25% aqueous KCl 25:20:20:20:17 (v:v) as the mobile phase at 20°C. The chromatogram was dried for 10 min at 120°C, cooled to room temperature and very lightly sprayed with a resorcinol reagent [0.2ml 0.1M CuSO₄, 5mL resorcinol (2% in water) and 40mL 8-N HCl]. The freshly sprayed chromatogram was covered with a hot (120°C) and clean 10 x 20cm glass-plate and immediately exposed to 120°C for exactly 3.5min in an air-ventilated oven. Pre-heating of the cover-plate (120°C) prevented undesirable condensation of the resorcinol reagent during thermal exposure of the chromatogram. GS appeared as blue-tinted spots against white background.

The above mentioned bovine-brain GS, analytical standards and references in the literature were used to tentatively attribute identity to the resorcinol-detected sialic acid chromophores on HPTLC plates.

A Desaga CD-60® (Heidelberg, Germany) scanning photodensitometer was used to measure the chromogenic response of GS spots visualized on HPTLC plates with a resorcinol reagent. Optical density of the detected GS spots was measured in reflectance mode at λ=570nm. This wavelength was separately determined as summarized below.

A λ=500 to λ=650nm absoption spectrum was measured using GD3 after its HPTLC migration in the carrier silica-gel stationary phase and resorcinol-mediated detection. The spectrum was measured against a GS-free background on the same HPTLC plate. The carrier silica gel medium caused a minor shift in the absorbtion maximum of sialic acid-resorcinol chromophore from λ=580 to λ=570nm. This was 10nm less compared to the absorbtion maximum (λ=580) of the same chromophore dissolved in a colorimetric solution (without silica gel).

### Colorimetry of Individual of GS Classes

Solutions of purified GS fractions containing 160nmol total sialic acid were quantitatively deposited as 2 x 160-mm bands on 10 x 20 cm Whatman HP-K silica-gel plates. In addition, these GS solutions were also deposited as 2 x 5mm reference bands on the same plate, i.e. its two 20-mm unloaded side lanes. The loaded HPTLC plate was chromatographed to separate GS into their individual classes as described above. In particular, the analyzed GS included those classes that were either contaminated by yellow-tinted, non-sialic acid chromophores or, for which standards did not exist. The reference side lanes of GS were visualized with resorcinol. Undetected individual GS classes that migrated between the reference bands were separately scrapped off and evaluated by colorimetry. Total sialic acid was determined using the same calibration curve as above.

### RESULTS AND CONCLUSION

The HPTLC densitogram of standard GS and GS isolated from Pakistan buffalo's mature milk is given in Figure 1. While the GS from buffalo milk display a very high peak that corresponds to the standard GD3, there are three further peaks (**1-3**). One of them (denoted **1**) is close to the standard GM1 and is likely to be a different species of GM1. The other two peaks are supposed to represent *O*-acetyl-GD3 (**2**) and a GT ganglioside (**3**), respectively.

In conclusion, GS isolated from Pakistan buffalo milk comprise a GM1 species, which is absent in bovine milk.

Table 1 compares the concentration of GM1 and total sialic acid derived from the example with the corresponding value of bovine milk (derived from the literature).

It can be seen from Table 1 that the GS allocated in Figure 1 to the GM1 class was is almost ten times more concentrated in buffalo than in bovine milk. The table also shows that important amounts (42%) of dairy GS partition into the organic phase (LP) of the Folch wash that contains hydrophobic lipids. This finding contrasts with the fact that published data on dairy GS were obtained by analysing the aqueous phase only in which the water-soluble GS are expected to accumulate.

**Table 1:**

| Quantitative data on sialic acid and GS in bovine and buffalo milk. | | |
|---|---|---|
| **Liquid Milk (1 kg)** | **Bovine Milk**^{**1)**} | **Mature Buffalo**^{**2)**} |
| **GM**_{**1**} **(nmol)** | 4.1 | 36.3 |
| - µ*g (MW 1569)* | *6.5* | *57.0* |
| **Total sialic acid (µmol)** | 8.2 | 6.8 |
| - *mg (MW 309.3)* | *2.5* | *2.1* |
| **Total sialic acid (%)** | | |
| *- in Folch's Upper* | *78* | *55* |
| *Phase* | | |
| *- in Folch's Lower* | *22* | *45* |
| *Phase* | | |

| | | |
|---|---|---|
| 1) Pooled fresh bovine milk from a farm in Switzerland | | |
| 2) Pooled fresh milk from two Pakistan Buffalo. | | |

### Example 2: Isolation and Identification of GS from Italian Buffalo Milk.

### MATERIALS AND METHODS

2000 g of Italian buffalo milk were obtained from an Italian breeder. The sample was delactosed by dialysis and partitioned into Folch upper (UP) and lower (LP) phase, according to procedure of Example 1. The phases were separated into acidic and non-acidic lipid fractions by ion-exchange chromatography (the upper phase was additionally dialysed to remove non-lipid contaminants). The acidic lower phase fraction was deacylated to eliminate acidic glycerophospholipids. The GS were purified with Unisil® in mini-columns (see Example 1). The same procedure as in Example 1 was applied.

GS were separated by bi-dimensional HPTLC in order to identify GM1 and unknown GS classes. A mixture of standard bovine brain GS was used as a reference for identification purposes. A 10 x 20cm HPTLC silica gel plate was cut to 10 x 11.5cm and eluted in two directions with chloroform-methanol 1:1. After this clean-up procedure, the plate was dried for 15min at 100°C. Figure 2 provides complementary details on loading of GS samples and their elution 1 and elution 2 as described below.

Elution 1: five-µl aliquots of the acidic upper and lower phases were deposited as a 2x2mm band onto the plate in the bottom-right comer of the plate (sampling zone 1). The total amount of deposited lipid-bound sialic acid was 2.9nmol. Furthermore, a first sample comprising six standard GS was deposited on the bottom-left corner. After the first elution using an acidic mobile phase methyl acetate-n-propanol-chloroform-methanol-0.25% aqueous KCl 25:20:20:20:17 (v:v), the plate was flushed with nitrogen to remove solvents, and turned 90° clockwise around the sampling zone 1 as an axis. Elution 2: a second sample comprising the same six standard GS was deposited on the bottom-right corner of the plate (sampling zone 2). In addition, three aliquots containing 64, 96 and 128pmol pure standard GM1 were separately and quantitatively deposited between the sampling zones 1 and 2. The elution of GS in the second direction was accomplished using an alkaline mobile phase chloroform-MeOH-2.5M aqueous ammonia-0.25 % KCl [50:40:10 (v:v)], after which the plate was dried as above.

As already mentioned earlier, GS are acidic lipids. Therefore, their HPTLC mobilities are usually different in acidic compared to alkaline mobile phases. GM1 and GD3 produce the most striking migration differences as follows. Elution 1: GM1 migrates ahead of GD3 due to the presence of an acidic mobile phase such as described earlier. Elution 2: the opposite occurs in an alkaline mobile phase (described earlier), in which GD3 migrates ahead of GM1. This unique migration pattern shown in Figure 2 is particularly useful for the identification of GM1 and GD3 using bi-dimensional HPTLC.

After elution, the chromatogram was flushed with a stream of nitrogen to remove solvents. GS were visualized using a resorcinol agent (see Example 1).

Figure 2 also shows the resorcinol-mediated chromogenic response of pure standard GM1, the accurate concentrations of which were 64-, 96- and 128-pmol. These visible GM1 spots align with that in the GS from buffalo milk chromatographed on the same plate. These four GM1 spots were evaluated by HPTLC scanning densitometry using a Desaga CD-60® apparatus (Desaga AG, Heidelberg, Germany) set to a λ=570-nm wavelength. Quantitative densitometric responses were obtained for the three standard GM1 spots and that detected in the GS from buffalo milk. The densitometric response of standard GM1 were plotted against their pmol concentrations to establish a calibration curve. The concentration of GM1 was determined using the above standard calibration curve and the densitometric data of GM1 that occurs in buffalo milk GS.

### RESULTS AND CONCLUSION

The bi-dimensional HPTLC chromatogram is depicted in Figure 2. A spot corresponding to the standard GM1 can be seen. Quantitative scanning densitometry revealed that Italian buffalo milk (1kg) contained about 25nmol (39µg) GM1. It was calculated that 0.3% of total lipid-bound sialic acid in Italian buffalo milk stems from GM1.

Figure 2 shows the presence of two major GS classes in Italian buffalo milk coded "K" and "L". These classes are highly polar (L is more polar than K) and as such migrating in the vicinity of QG-1b, which is a tetrasialo-GS found in bovine brain (not shown in Figure 2). The sialic acid content of these highly polar GS accounted for 26% of the total lipid bound sialic acid in Italian buffalo milk. These two GS are so far not yet described GS and are therefore novel.

Table 2 lists concentrations of GM1 and polysialo-GS derived from Examples 1 and 2 (Pakistan and Italian buffalo milk) and values obtained from the literature for bovine and human milk types.

It is found that human and buffalo milk contains similar levels of GM1 and polysialo-GS. Human milk has been shown to comprise 0.3 to 2.6% of total sialic acid in the form of GM1 (buffalo and bovine milk: 0.3% and up to 0.05%, respectively) and 8 to 28 % of total sialic acid in the form of polysialo-GS (buffalo and bovine milk: 26% and traces, respectively).

This makes buffalo milk an ideal source for humanisation of soy- or cow milk-derived baby-or infant formulas, or for replacing cow milk solids by buffalo milk solids in these formulas.

**Table 2:**

| Gangliosides in mammalian milk types. | | | |
|---|---|---|---|
| **Liquid Milk** | **GS-Bound Sialic Acid (NeuAc)** | | |
| | **mg/L** | **% of Total NeuAc** | |
| | | **Ganglioside** **GM1** | **Polysialo- Gangliosides** |
| Human Colostrum | 2.2 to 3.6 | 0.6 to 1.4 | 13 to 29 |
| Human Milk | 2.1 to 4.3 | 0.3 to 2.6 | 8 to 28 |
| Bovine Milk | 2.7 | 0.05 | Traces |
| Buffalo Pakistan | 2.1 to 3.3 | 0.1 to 0.5 | Traces |
| Buffalo Italy (28% TS) | 2.5 | 0.3 | 26 |
| Buffalo Serum Italy | 0.8 | 0.3 | 26 |

### Example 3: Gangliosides Isolated from Italian Buffalo Milk Serum

On the basis of the analysis of a 3kg sample of liquid Italian buffalo milk serum commercially obtained from a Mozzarella cheese producer in Italy an extrapolation was performed to calculate values of 1000kg. Accordingly, the milk serum was delactosed and desalted and the retentate comprised 9880g dry matter. One g of the retentate contains 97µg lipid-bound sialic acid.

It was calculated that 17g delactosed and powdered Italian buffalo milk serum equate in lipid-bound sialic acid to 1.0L human milk. It was found by isolating GM1 from the serum that again 0.3% of total lipid-bound sialic acid is found in the form of (or bound to) GM1, which compares to the levels found in human milk. In absolute amounts this translates to 31µg GM1 in 17g delactosed and powdered Italian buffalo milk serum. Thus, 1.0L of soy or cow milk may be humanized by adding 17g delactosed and powdered Italian buffalo milk serum.

Italian Buffalo milk serum, especially if delactosed, is therefore suitable for directly applying to nutritional formulas for babies or infants in order to adjust the GS level of the formula to the corresponding levels of human milk.

Furthermore, the serum, which is a readily available byproduct of Mozzarella production in Italy is a suitable source for obtaining GS, in particular GM1, polysialo-GS and/or unknown GS.

### Example 4 : Binding of Cholera Toxin B-Subunit by Italian Buffalo GM-1.

The binding of GM1 isolated from Italian buffalo milk serum to the B-subunit of cholera toxin (CTB) was visualised by a HPTLC-overlay assay. The method is detailed by Cambron-LD and Leskawa-KC [(1990) *A sensitive method to quantitate gangliosides of the gangliotetraose series directly on chromatograms using peroxidase conjugated cholera toxin,* Stain Technology 65: 293-297]. GM1 was isolated according to the methodology of Example 1 from Italian buffalo milk serum (Example 3).

Each of two identical series "A" and "B" consisted of two samples: 1) bovine brain-GS; 2) the serum-derived monosialo-GS that migrate in the GM1 region on HPTLC plate. Series "A" and "B" were deposited on four separate lanes using aluminium foil-backed silica gel plates. After elution, the "A" series on the foil was cut off from the "B" series using scissors. The part of the foil including the "A" series was treated with an anisaldehyde reagent to visualize any lipid-bound sialic acid, that is all GS and hematosides. They appeared as blue-tinted spots against white background as shown in Figure 3 and lanes 1 and 2. Figure 3 shows only a magnified GM1 plus GD1a section of the whole chromatogram. The part of the foil including the series "B" was treated with a polyisobutylmethacrylate (Aldrich No. 18155-2) solution to immobilize GS in the chromatographic sorbent. The foil containing immobilized GS was further exposed to a CTB solution. Unbound CTB was washed off, whereas CTB that remained bound to GS was visualized using a chlorophenol reagent. This GS-bound CTB is shown in the right section of the chromatogram in lanes 3 and 4. This figure clearly shows that bovine-brain GM1 species and those present in the GS from Italian buffalo milk serum bind CTB. The same detection procedure was applied to GS from bovine milk to detect GM1 (not shown). In this case only the standard bovine-brain bound CTB. This demonstrated that GM1 in bovine-milk GS occurs at levels that are below the limits of detection with respect to the above described method.

It was found that the GM1 isolated from buffalo milk serum binds to cholera toxin subunit B, similar to bovine brain GM1. In contrast, a disialo-GS from bovine brain (GD1a) was not capable of binding the toxin.

In summary, the GM1 isolated from buffalo milk exhibits the functionality of bovine brain GM1. It may be inferred that GM1 from buffalo milk is as efficient in preventing toxin binding to small intestine cells as other GS species of the GM1 class. The GM1 from buffalo milk may prevent symptoms and infection of the small intestine originating from *Vibrio cholerae*.

Most likely, GM1 from buffalo milk will also share other functional properties of so far described GM1 species, including binding to various toxins.

### Example 5: Anti-Inflammatory Effects of Buffalo Milk Gangliosides.

The inflammatory response to tumor necrosis factor (TNFα ) in a cell culture model is determined by measuring PGE-2 production (Enzyme-Linked Immuno Assay = ELISA) in the milieu of cell culture as an outcome. The anti-inflammatory property of bioactive molecules is assessed by their inhibitory effects on PGE₂ production.

Non-tumorigenic human colonic hepithelial (HCE) cells, were inoculated into culture dishes and grown in serum free medium. GS isolated either from Italian buffalo milk, from Italian buffalo milk serum or bovine milk, wherein the same amount of lipid-bound sialic acid was applied (5 and 10nmoles/ml), were added to the cell culture 30h prior to cell stimulation with TNFα (10ng/ml). The milieu is then collected for PGE₂ measurements, whereas the cells are washed with PBS and total protein determined.

GS derived from the buffalo milk and milk serum strongly inhibited PGE-2 secretion (Figure 3) mediated by the pro-inflammatory agent.

In conclusion, GS isolated from buffalo milk prevent inflammatory reaction of intestinal cells as measured by PGE-2 production. They are suitable to inhibit PGE-2 secretion and to down-regulate inflammatory reactions in the intestines.

### Example 6 : Infant Formula Comprising Gangliosides derived from Buffalo Milk

An infant formula based on Italian buffalo milk serum was simulated by mixing powdered Italian buffalo milk serum protein (1.4g/dl) with vegetable oils (3.6g/dl) and lactose (7.1g/dl). The formula was further enriched with iron, zinc and copper (0.7, 0.7 and 0.06mg/dl, respectively). Taurine was added at a similar level to that of breast milk.

The exact amino acid composition of the protein fraction isolated from buffalo milk serum was so far not analysed. This and further analysis need to be done prior to potential feeding of new-borns, babies and infants.

However, with respect to the GM1 and polysialo-GS profile, the formula is more similar to that of human breast milk than a formula based on cow's milk.

## Claims

1. Gangliosides (GS), **characterised in that** they are obtainable, obtained or derived from buffalo milk, skimmed buffalo milk, buffalo milk serum, and/or derivatives of either.

2. GS according to claim 1, **characterized in that** they are isolated GS.

3. GS according to any of claims 1 or 2, wherein the buffalo milk serum or the derivative of it is buffalo milk serum obtained from mozzarella or ricotta cheese production.

4. The GS according to any of claim 1 to 4, which are functional equivalents from GS present in human tissue and/or secreted fluids including milk.

5. GS according to any of claim 1 to 5, **characterised in that** they are present in or isolated from the upper aqueous and/or the lower organic phase according to Folch.

6. The GS according to c any of claim 1 to 5, wherein the GS belong to the classes of GM1, GM2, GM3, GD3, GT-1a, GT-1b, branched GT and/or unknown GS defined as "GS-L" or branched "GM-F".

7. The GS according to any of claim 1 to 6, wherein the GS exhibit a mono-, or bi-dimensional HPTLC migration pattern that allows or suggests allocation of the GS to at least one GM, GD or GT occurring in human milk.

8. The GS according to any of claim 1 to 7, for use as a composition and/or supplement in food products, instant milk, infant and/or other nutritional formulas, and/or pet food.

9. The GS according to any of claim 1 to 8, for use as an anti-inflammatory agent.

10. The GS according to any of claim 1 to 9, for use as a binding and/or neutralising agent of infective and/or toxic agents.

11. GS according to any of claim 1 to 10, obtainable by
delactosing buffalo milk, skimmed buffalo milk, buffalo milk serum or derivatives of either,
partitioning lipids in organic phases,
isolating acid lipid fractions, and/or, optionally
separating GS from other acid lipids.

12. A composition enriched with GS, wherein the composition comprises fractions isolated from buffalo milk.

13. The use of buffalo milk, skimmed buffalo milk, buffalo milk serum and/or derivatives of either as a source of GS.

14. The use of buffalo milk, skimmed buffalo milk, buffalo milk serum and/or a derivative of either to humanize infant or nutritional formulas or for isolation of GS that are functionally equivalent to GS occurring in humans.

15. GS, **characterized in that** they are present in or isolated from the lower phase according to Folch and **in that** they are not present in the upper aqueous phase.
